# EUROPÄISCHE PATENTSCHRIFT

(11) **EP 2 467 721 B1**
(45) Veröffentlichungstag und Bekanntmachung des Hinweises auf die Patenterteilung: **30.12.2020**
(21) Anmeldenummer: 10739645.9
(22) Anmeldetag: 06.08.2010
(51) Int. Cl.: G01N 33/543, G01N 33/558

(54) **TESTSYSTEM ZUR VISUELLEN AUSWERTUNG**
TEST SYSTEM FOR VISUAL ANALYSIS
SYSTÈME DE TEST PERMETTANT UNE ÉVALUATION VISUELLE

(30) Priorität: 17.08.2009 DE 102009037791
(43) Veröffentlichungstag der Anmeldung: 27.06.2012
(73) Patentinhaber: DST Diagnostische Systeme & Technologien GmbH, 19059 Schwerin (DE)
(72) Erfinder: VON OLLESCHIK-ELBHEIM, Lars, 48151 Münster (DE); HÜNKEN, Mark, 19061 Schwerin (DE); DANGERS, Marc, 19055 Schwerin (DE); PUTENSEN, Olaf, 19055 Schwerin (DE)
(74) Vertreter: Simandi, Claus
(86) Internationale Anmeldenummer: PCT/EP2010/061525
(87) Internationale Veröffentlichungsnummer: WO 2011/020724

(56) Entgegenhaltungen:
- WO-A1-02/088739
- WO-A1-2007/063423
- WO-A1-2007/122403
- US-A- 4 943 522
- US-A1- 2003 119 203
- Babakhin AA et al.: "(PDF) Assessment of FastCheckPOC (second generation): a new rapid, in vitro test for assessment of sensitisation to inhalant allergens", , 1 January 2010 (2010-01-01), XP055547288, Retrieved from the Internet: URL:https://www.researchgate.net/publicati on/281106026_Assessment_of_FastCheckPOC_se cond_generation_a_new_rapid_in_vitro_test_ for_assessment_of_sensitisation_to_inhalan t_allergens [retrieved on 2019-01-24]

## Beschreibung

Die vorliegende Erfindung betrifft ein Testsystem zur visuellen Auswertung und dessen Verwendung im Point-of-Care Bereich.

In der Forschung, Diagnostik, oder einer Vielzahl weiterer Anwendungsfelder stellen analytische Labortests, die zur qualitativen oder/und quantitativen Bestimmung von Molekülen, Analyten bzw. deren Aktivität oder Zusammensetzung dienen, die Basis für weitreichende Aussagen bis hin zur Entwicklung neuer Verfahren oder Vorrichtungen dar. Die Basis sind die allgemein bekannten Methoden der DNA/RNA Analytik bzw. der Proteinanalytik. Ein weiteres Beispiel sind die Vielzahl von analytischen Verfahren und Methoden, die eingesetzt werden, um die Antikörperreaktionen, sogenannte Immunreaktionen, welche für die Bestimmung von (Bio)Markern und vielen weiteren Substanzen/Analyten eingesetzt werden.

Es sind Schnelltestverfahren bekannt, wie der Lateral-Flow-Test (LFT), Flow-Through-Test (FTT), Agglutinations-Test (AT) oder Solid-Phase-Test (SPT). Alle diese Verfahren dienen zum schnellen Nachweis von Analyten ohne die Verwendung von Geräten und sind zur visuellen Auswertung geeignet.

Ein robustes Assayprinzip ist bekannt, wie im Stand der Technik zur in-vitro Diagnostik als Immunoassay (IA), insbesondere als EIA, oder "binding assay" ("sandwich") beschrieben (Siehe z.B. EP0171150B1, EP 0063810B1). Ferner ist z.B. der fluorometrische Nachweis von IgE im Rahmen eines Bindungsassays aus EP0119613 B1 bekannt. Darüber hinaus, sei auf das Schrifttum von Roger P. Ekins (z.B. WO 8401031 u.v.a.) verwiesen.

Ferner ist ein membrangestützer Bindungsassay, insbesondere von IgE aus Blut an Allergenen seit Ende der 1980-iger Jahre bekannt. Z.B. CHEMICAL ABSTRACTS, vol. 101, no. 25, 17th December 1984, page 578, abstract no. 228190b, Columbus, Ohio, US; B.J. WALSH et al.: "Allergen discs prepared from nitrocellulose: detection of IgE binding to soluble and insoluble allergens", & J. IMMUNOL. METHODS 1984, 73(1), 139-45 oder in Form eines Teststreifens (WO2007/063423 A1).

US 4 943 522 A offenbart einen Teststreifen zur visuellen Auswertung der Bindung eines Analyten an einen in einer Indikatorzone gebundenen spezifischen Rezeptor mittels eines Detektionsreagens, wobei in der speziellen Ausführung gemäß Beispiel 6 und Abb. 9 mehrere Indikatorzonen mit unterschiedlichen Rezeptor-Konzentrationen eine semiquantitative Testdurchführung ermöglichen.

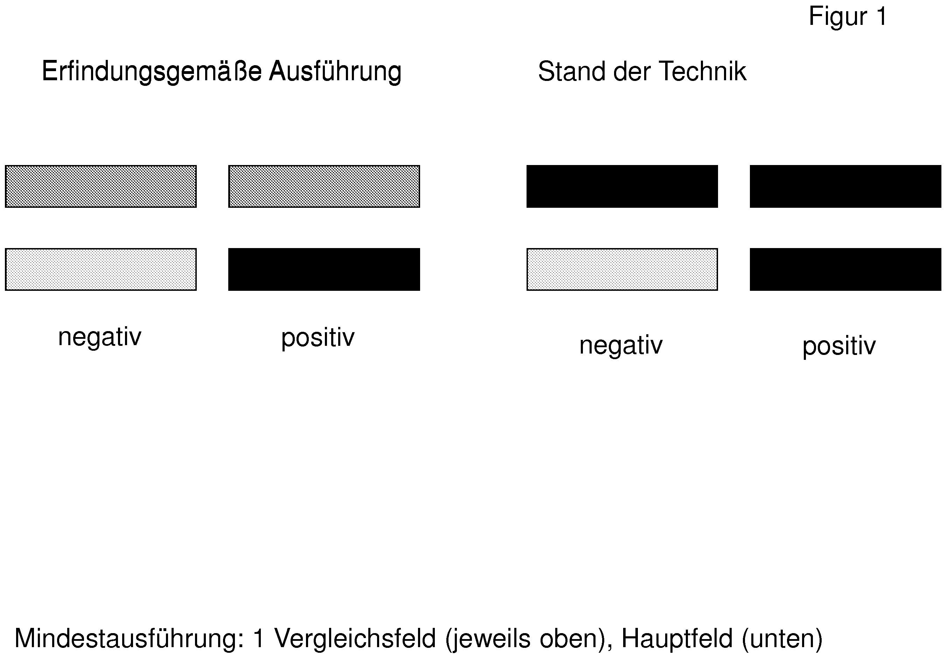

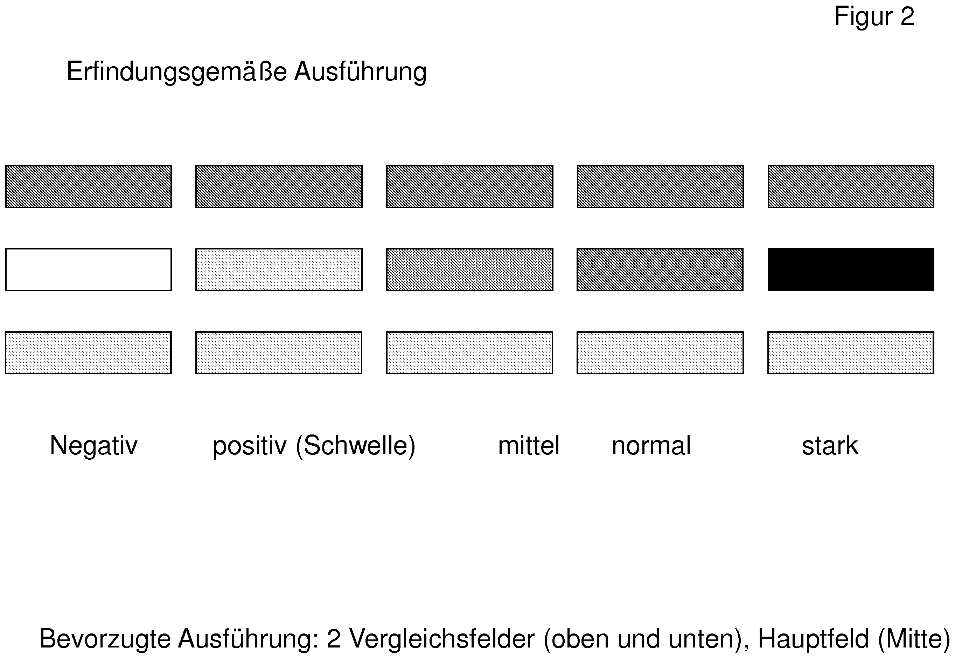

WO 02/088739 A1 lehrt einen Teststreifen zur visuellen Auswertung der Bindung eines Analyten an einen in einer Indikatorzone gebundenen spezifischen Rezeptor mittels eines Detektionsreagens, wobei in der speziellen Ausführung gemäß Abb. 2 drei Indikatorzonen mit unterschiedlichen Analytkonzentrationen reagieren und somit eine semiquantitative Testdurchführung erlauben.

US 2003/119203 A1 offenbart einen Teststreifen zur visuellen Auswertung der Bindung eines Analyten (z.B. eines Allergens) an einen in einer Testzone gebundenen spezifischen ersten Rezeptor mittels eines Detektionsreagens, wobei sich auf dem Teststreifen ein Kalibrierungsbereich mit weiteren Kontrollzonen befindet, die mit unterschiedlichen Konzentrationen eines zweiten Rezeptors belegt sind und eine semiquantitative Testdurchführung ermöglichen.

Für den Point-of-Care (POC) Bereich ist beispielsweise der käuflich erhältliche Fast-Check-Poc der Anmelderin beschrieben (siehe EP1718970), der auf Basis eines membrangestützen Bindungsassays zum Allergienachweis aus Vollblut eingesetzt werden kann.

WO 2007/122403 A1 beschreibt einen Point-of-Care Teststreifen zur visuellen Auswertung der Bindung von hCG an einen in einer Testzone gebundenen hCG-Antikörper mittels eines Detektionsreagens, wobei mehrere Testzonen mit Hilfe von unterschiedlichen Antikörperverdünnungen eine semiquantitative Testdurchführung erlauben.

Es besteht jedoch ein hohes Bedürfnis, die Aussagekraft eines visuell auslesbaren Testsystems zu verbessern.

Ein besonderes Problem ist die Schwierigkeit, allein mit dem Auge und ohne weitere technische Hilfsmittel einen Intensitätskontrast mit mehr Graustufen als "hell" und "dunkel zuverlässig zu bewerten. Dies ist im Point-of Care unerlässlich, da ein schnell auswertbares Ergebnis ohne weitere Hilfsmittel (z.B. Reader, Sehhilfen etc.) gewährleistet sein muss.

Es sind mehrere Testsysteme käuflich erhältlich, die auf dem Prinzip des Lateral-Flow Assays beruhen (z.B. Phadia Rapid (www.phadia.com)). Bei diesem Test werden die im Blut befindlichen Antikörper gegen spezifische Antigene dadurch nachgewiesen, dass ein Antigen auf einem Nachweisstreifen auf einer Trägermembran fixiert ist, die Antikörper aus der Probe an diesem Antigen haften und ein markierter Antikörper an diesem Antikörper aus der Probe bindet. Die Markierung, in diesem Beispiel mit Gold-Nanopartikeln, lässt den Streifen farbig erscheinen, sobald ausreichend markierte Antikörper gebunden haben. In der Nähe des Nachweisstreifens befindet sich ein Kontrollstreifen, auf dem ein Gemisch verschiedener humaner IgE fixiert ist. Der markierte Antikörper bindet daher am Kontrollfeld, jedoch unabhängig vom Vorhandensein spezifischer IgE in der Probe und daher immer, sofern Membranstreifen und markierter Antikörper funktionstüchtig sind.

Der Fast-PoC Check Test der Anmelderin (supra, EP1718970) basiert auf einem fixierten Antigen, an das Antikörper aus einer Probe binden. Nach einem Waschschritt wird ein weiterer, mit dem Enzym alkalische Phosphatase markierter Antikörper zugegeben, der an den ersten Antikörper bindet. Nach einem weiteren Waschschritt wird ein Substrat für das Enzym zugegeben, das sich bei seiner Umsetzung verdunkelt, so dass ein dunkler Streifen auf dem Nachweisplatz entsteht. Diese Nachweisreaktion wird mit einer Stopplösung angehalten, um zu verhindern, dass allein die geringe Anzahl unspezifisch gebundener, markierter Antikörper schon zu einer Dunkelfärbung führt.

Weiter Beispiele sind das CLA System von Hitachi (http://www.hcdiagnostics.com/CLAIntnl.html), und das Allergenscheibensystem von Dr. Foooke (http://www.fooke-labs.de/inVitro/allergie_diagnostik_1.htm).Sie alle beruhen auf einer visuellen Erfassung eines sich verfärbenden oder verdunkelnden Streifens oder anders geformten Probenträgers.

Keines der Verfahren erlaubt jedoch eine Quantifizierung über die beiden Werte "hell" und "dunkel" hinaus, da das menschliche Auge nicht in der Lage ist, absolute Helligkeitswerte objektiv zu bestimmen.

Der von Hoffmann-LaRoche (CH) vertriebene Test "Combur" und ähnliche Urin-Streifentests dagegen unterscheiden sich von den vorgenannten Tests, da er eine Vielzahl von Testfeldern umfasst. Sie sind auf unterschiedlichen Assayprinzipien aufgebaut, allen gemeinsam ist jedoch eine Änderung der Farbintensität von farblos / weiß zu Farbintensiv, die visuell ausgewertet werden können. Dazu sind farbige Flächen auf der Verpackung des Combur-Tests aufgedruckt, mit Hilfe derer der Anwender die Stärke der Verfärbung aus dem Test einschätzen und klassifizieren kann.

Ein Nachteil dieses Verfahrens ist jedoch, dass ein gedrucktes Vergleichsfeld keine Aussage über einen ordnungsgemäßen Verlauf des Tests und keine Kontrolle liefert. Wenn erwünscht, muss diese Information über weitere Kontrollfelder geliefert werden, was die Kapazität des Teststreifens bezüglich der Anzahl auslesbarer Tests reduziert.

Daher ist es Aufgabe der vorliegenden Erfindung, für ein Testsystem eine verbesserte visuelle Auswertung zu ermöglichen.

Die Aufgabe wird dadurch gelöst, dass das Testsystem für den visuellen Betrachter unterschiedliche Helligkeiten erzeugt, welches eine vorteilhafte sichere Auswertung ermöglicht.

Daher betrifft die Erfindung ein Testsystem zur visuellen Auswertung, vorzugsweise für das bloße Auge ohne weitere Hilfsmittel, die eine Auswertungseinheit eines Allergietests oder Nahrungsmittelunverträglichkeitstests aufweist.

Diese Auswertungseinheit eines Allergietests oder Nahrungsmittelunverträglichkeitstests für eine visuelle Auswertung eines Testergebnisses besteht aus mindestens zwei optisch unterscheidbaren Feldern,
wobei mindestens ein erstes Hauptfeld Rezeptormoleküle einer Spezies aufweist,
wobei mindestens ein Allergen Rezeptormoleküle einer Spezies ist,
und mindestens ein weiteres Vergleichsfeld eine geringere Dichte an Rezeptormolekülen einer Spezies aufweist,
wobei sämtliche Rezeptormoleküle einer Spezies auf einem Träger fixiert sind,
wobei die Felder für eine Probenflüssigkeit enthaltend einen oder mehrere Binder benetzbar sind und der Bindungserfolg mit einem Detektionsmittel nachgewiesen wird, so dass die Auswertungseinheit unterschiedliche Helligkeiten (z.B. Graustufen) erzeugt.

Zur Herstellung von Feldern mit geringerer Dichte können die Rezeptormoleküle relativ 10 bis 90 Gew. %, insbesondere 20 bis 80 Gew. % zum Hauptfeld, welches 100 % Gew % an Rezeptormolekülen aufweist, betragen.

Diese erfindungsgemäße Auswertungseinheit erlaubt eine sichere visuelle Auswertung mit dem bloßen Auge und macht sich den Umstand zunutze, dass das Auge zwar keine absoluten Helligkeiten bestimmen, jedoch relative Helligkeitsunterschiede erkennen kann.

Besonders vorteilhaft erlaubt die erfindungsgemäße Auswertungseinheit daher das Erkennen von relativen Schwellenwerten für den jeweiligen Binder/Analyten an ein Rezeptormolekül in einer Auswertungseinheit.

In einer weiteren Ausführungsform sind die optisch unterscheidbaren Felder auf einer Auswertungseinheit räumlich voneinander getrennt. Dies kann z.B. durch Aussparungen auf dem Träger erreicht werden oder in jeder anderen Weise erfolgen.

Weiterhin ist bevorzugt, dass ein oder mehrere Hauptfelder von mindestens zwei (Vergleichs-)Feldern mit geringerer Dichte an Rezeptormolekülen in einer Auswertungseinheit umgeben ist. Die Vergleichsfelder haben untereinander bevorzugt jeweils eine unterschiedliche Dichte. Beispielsweise sind bei zwei Vergleichsfeldern je 10 und 90%, 20 und 80% oder 30 und 70% Dichte eingestellt.

In einer weiteren Ausführungsform kann das Vergleichsfeld eine verschiedene oder gleiche Spezies Rezeptormoleküle als das Hauptfeld aufweisen. Maßgeblich ist jedoch, dass mindestens ein Binder im Hauptfeld und / oder Vergleichsfeld an ein Rezeptormolekül in einer Auswertungseinheit bindet.

Bleibt z.B. das Hauptfeld in seiner Helligkeit unverändert und wird das Vergleichsfeld in seiner Helligkeit verändert, ist der Test negativ.

Im Fall eines Allergietest oder Nahrungsmittelunverträglichkeitstest kann das Rezeptormolekül ein Antigen sein, während die Vergleichsfelder z.B. Rezeptormoleküle einer Spezies aufweisen, die aus einer anderen Quelle, insbesondere aus Blutplasma, gewonnen werden, oder synthetisch hergestellt werden. Insbesondere können dies Antikörper, und insbesondere Immunglobuline, wie IgE, IgA1, IgA2, IgA3, IgA4, IgG oder IgG1 - 4 sein.

Von daher kann ein Rezeptormolekül in einem Vergleichsfeld mit einem Binder identisch sein, der in das Hauptfeld an die Rezeptormoleküle bindet.

Daher betrifft die Erfindung in einer weiteren Ausführungsform eine solche, wobei mindestens ein Vergleichsfeld ein Rezeptormolekül einer Spezies aufweist, der mit einem Binder für ein Rezeptormolekül im Hauptfeld identisch ist.

Es ist weiter in dieser Ausführungsform bevorzugt, dass ein Detektionsmittel an den Binder des Vergleichsfelds und den bei positivem Ergebnis vorhandenen identischen Binder des Hauptfelds bindet und die Änderung der Helligkeit oder der Farbe hervorruft. Insbesondere kann das Detektionsmittel ein sekundärer Antikörper gegen den primären Antikörper (=Binder), insbesondere gegen Immunglobuline, wie IgE, IgA1, IgA2, IgA3, IgA4, IgG oder IgG1 - 4 sein. Der sekundäre Antikörper (=Detektionsmittel) ist bevorzugt markiert mit Gold-Nanopartikeln, Quantum Dots, oder fluoreszierenden Partikeln, unter denen insbesondere Fluoreszenzfarbstoffe bevorzugt sind, oder mit Enzymen wie der alkalischen Phosphatase, die mit einem Substrat reagiert und so eine Helligkeits- oder Farbänderung hervorruft. In einer weiteren Ausführungsform enthält die Auswertungseinheit ein Kontrollfeld, wobei unabhängig von der Probenflüssigkeit eine Helligkeit erzielt wird. Hierbei wird durch das Detektionsmittel eine Helligkeit erzielt. Dies kann z.B. ein Feld sein, wobei das Rezeptormolekül mit einem Binder abgesättigt ist. Ein solches Kontrollfeld kann daher eine Positiv- oder Negativkontrolle sein.

Eine weitere bevorzugte Ausführungsform betrifft eine Anordnung, wobei das Hauptfeld von einem Kontrollfeld und einem Feld geringerer Dichte umgeben ist.

Eine solche Ausführungsform ist beispielsweise eine Anordnung von Balken, die zudem optisch voneinander unterscheidbar sind, da die Balken Zwischenräume (Aussparungen)aufweisen (siehe Figuren).

In einer besonders bevorzugten Ausführungsform sind mehrere erfindungsgemäße Auswertungseinheiten mit gleichen, vorzugsweise jedoch unterschiedlichen Rezeptormolekülen einer Spezies unter Ausbildung eines Testsystem auf einem Träger aufgebracht, vorzugsweise in Form eines Koordinatensystems angeordnet.

Dies erlaubt vorteilhaft die simultane Bestimmung mehrerer, insbesondere mehr als 5 und insbesondere mehr als 8 Rezeptormoleküle unterschiedlicher Spezies in unabhängigen Auswertungseinheiten mit einer Probenflüssigkeit.

Die erfindungsgemäße Auswertungseinheit wird zur Diagnose von Allergien und Nahrungsmittelunverträglichkeit bei Tier und Mensch eingesetzt. Beispielsweise zur Bestimmung von Allergien, wobei Allergene solche Rezeptormoleküle repräsentieren und der Binder, wie Immunglobuline, direkt aus dem Blut nachgewiesen werden können.

In einer weiteren bevorzugten Ausführungsform ist die erfindungsgemäße Auswertungseinheit für ein Testsystem im Point-of-Care Bereich geeignet. Hierzu muss ein solches Testsystem in einer Kammer vorliegen. Weiterhin ist bevorzugt, dass die Ausmaße Taschenformat aufweisen und das Testsystem eine Länge bis zu 20 cm, Höhe bis zu 10 cm und eine Breite von bis zu 20 cm aufweist. Diese Größen erlauben einen handlichen und mobilen Umgang, so dass im Point-of-Care Bereich eine schnelle Auswertung erfolgen kann.

Im Rahmen dieser Erfindung wird unter Point-of-Care verstanden:
- Durchführung von Laboruntersuchungen in unmittelbarer Nähe zum Patienten, außerhalb eines Zentrallabors;
- Keine Probenvorbereitung, insbesondere keine Pipettierarbeiten, das Untersuchungsmaterial ist vorzugsweise Vollblut;
- Einsatzbereite Reagenzien (ready-to-use), etwa in Tank- oder Kassettenform;
- Messgeräte, die nur für Einzelprobenmessung vorgesehen sind;
- Keine eingehende medizinisch-technische Weiterbildung zur Nutzung notwendig;

Daher betrifft die Erfindung ebenfalls die Verwendung des erfindungsgemäßen Allergietests oder Nahrungsmittelunverträglichkeitstests im Point-of-Care Bereich.

Weiterhin betrifft die Erfindung ein entsprechendes Verfahren zur Diagnose von Allergien und
Nahrungsmittelunverträglichkeit, wobei eine Probenflüssigkeit auf mindestens zwei optisch unterscheidbaren Feldern einer Ausleseeinheit aufgetragen wird,
wobei mindestens ein Allergen Rezeptormoleküle einer Spezies sind,
wobei mindestens ein Hauptfeld Rezeptormoleküle einer Spezies aufweist,
und mindestens ein weiteres Vergleichsfeld eine geringere Dichte an Rezeptormolekülen einer Spezies aufweist, wobei sämtliche Rezeptormoleküle einer Spezies auf einem Träger fixiert sind, wobei die Felder mit der Probenflüssigkeit enthaltend einen Binder benetzt werden, wobei der Binder mindestens ein Immunglobulin ist, und der Bindungserfolg mit einem Detektionsmittel nachgewiesen wird, so dass die Auswertungseinheit unterschiedliche Helligkeiten (z.B. Graustufen) erzeugt.

Weitere verfahrensgemäße Ausgestaltungen können den vorstehenden Ausführungsformen entnommen werden.

Die Begriffe "mit bloßem Auge" oder "visuell" werden synonym verwendet und bedeuten jeweils, dass die im Nachweis hervorgerufene Änderung der Helligkeit ohne weitere technische Hilfsmittel vom menschlichen Auge bestimmt werden kann, insbesondere ohne die Notwendigkeit eines Auslesegerätes.

Der Begriff "Feld" wird synonym für "Nachweisplatz" verwendet.

Der Auslesewert des erfindungsgemäßen Testverfahrens kann die Helligkeit eines Punktes oder einer Fläche sein, die sich beim Nachweis mittels Detektionsmittel derart ändert, dass sie visuell erkennbar ist. Dabei kann die Helligkeit bei Präsenz des Analyten erhöht oder vermindert werden. Unter "Helligkeit" kann auch eine Farbänderung verstanden werden. "Dunkelheit" ist ebenfalls das Ergebnis einer Helligkeitsveränderung.

Weitere Beispiele zur "Helligkeit" umfassen ebenfalls die Farbsättigung eines farbigen Testreagenz, eine Änderung der Farbe eines Testreagenz, die Änderung der Größe oder anderer flächiger Strukturelemente eines Punktes oder einer Fläche, die Änderung einer anderen optischen Materialeigenschaft wie Glanz, Transparenz, Körnigkeit einer Fläche. Kombinationen dieser Änderungen können bevorzugt sein. Daher können Helligkeitsveränderungen von "Weiss" über "Grau" zu "Schwarz" und umgekehrt erfolgen. Bevorzugt ist der sichtbare Träger zur Herstellung eines Kontrastes "Weiss" oder "Schwarz" oder entsprechend beschichtet.

Die Intensität eines Auslesewertes ist seine visuell erkennbare Eigenschaft oder Eigenschaften, die sich bei der Nachweisführung ändern.

Der Binder ist vorzugsweise ein Analyt und kann eine beliebige Substanz oder Substanzgemisch, ggfs. samt Lösungsmittel, beliebiger Herkunft sein, insbesondere von einer Pflanze oder einem Tier, bevorzugt einem Säugetier, und besonders bevorzugt von einem Menschen. Der Binder oder Analyt ist erfindungsgemäß Bestandteil einer Probenflüssigkeit, gereinigt oder rein, die jedoch vorzugsweise von einer Körperflüssigkeit stammt und vorbehandelt sein kann. Bevorzugt sind Körperflüssigkeit wie Vollblut, Halbblut, EDTA stabilisiertes Blut, Serum, Speichel, Tränenflüssigkeit, Urin oder Gehirnflüssigkeit. Im weitesten Sinne ist der Binder an die Rezeptormoleküle adressiert.

Die erfindungsgemäßen Felder befinden sich auf einem (Nachweis-)Träger, in so räumlicher Nähe, dass beide Felder beim visuellen Betrachten im selben Blickfeld liegen. Bevorzugt ist ein (Nachweis-)Träger mit mehr als einem Feld. Bevorzugt sind (Nachweis-)Träger, bei denen weitere, für den Nachweis sinnvolle Testreagenzien zugeführt werden können, beispielsweise mit einer Pipette oder Pumpe oder mit Kapillarkraft. Besonders bevorzugt sind Nachweisträger, auf denen Flüssigkeiten in Mikrokanälen zugeführt werden, wobei der Träger in einer Kammer hergerichtet ist.

Die Felder können sich direkt auf dem Substrat des Nachweisträgers befinden oder auf einer Beschichtung. Bevorzugt sind Gele oder Membranen, insbesondere Nitrozellulose-Membranen, auf oder in denen sich die Rezeptormoleküle bzw. Testreagenzien befinden. Bevorzugt ist mindestens ein Rezeptormolekül oder Testreagenz auf oder in der Membran fixiert, wie immobilisiert, gespottet, o.a.. Es kann bevorzugt sein, dass sich mehr als eine Membran auf dem Nachweisträger befinden, insbesondere dass sich jede Auswertungseinheit auf einer eigenen Membran befindet, bzw. ein Nachweisplatz und mindestens ein zugehöriger Vergleichsplatz, oder mehrere Nachweis- und Vergleichsplätze auf einer Membran. Ferner kann es bevorzugt sein, mehrere Auswertungseinheiten auf einer Membran zu positionieren, und davon getrennt mehrere Auswertungseinheiten auf einer anderen Membran.

Die Intensität der Auslesewerte ändert sich beim Nachweis sowohl auf einem Nachweisplatz wie auf den zugeordneten Vergleichsplätzen in die gleiche Richtung.

Es kann bevorzugt sein, dass der erfindungsgemäße Nachweisträger Strukturen enthält, die die visuelle Bestimmung erleichtern; insbesondere optische Elemente wie zum Beispiel transparente oder milchige Fenster, Linsen, Fresnellinsen, Prismen, Spiegel, Strahlteiler, Gitter, Hologramme oder andere digitale optische Elemente, die die direkte Betrachtung der Plätze ermöglichen. Die erfindungsgemäße Durchführung des Nachweises schließt auch mögliche, vom Anwender als vorteilhaft empfundene künstliche Beleuchtung ein, und Beleuchtung, die in einem bestimmten spektralen Bereich selektiv sein kann. Dieser spektrale Bereich kann den IR- oder UV-Bereich umfassen, die anregungsseitig visuell nicht direkt wahrnehmbar sind, im Nachweis jedoch einen Auslesewert im sichtbaren Bereich erzeugen.

Erfindungsgemäß ist die Fläche der Felder ausreichend groß, um die visuelle Bestimmung direkt oder mit Hilfe der integrierten optischen Elemente zu ermöglichen. Die Felder können rund, elliptisch, oval, dreieckig, polygonal, quadratisch oder rechteckig geformt sein, insbesondere in Form eines länglichen Streifens oder Balkens, oder ganz unregelmäßig. Er kann unterteilt sein, beispielsweise in zwei Streifen, oder ringförmig mit einem unveränderlichen Bereich im Inneren des Platzes.

Es kann vorteilhaft sein, einen Streifen mit sichtbaren Elementen zu strukturieren, insbesondere mit Streifen, die quer zu einem streifenförmigen Nachweisplatz liegen und bei positivem Nachweis als "+" wahrgenommen werden, und bei negativem Nachweis als "-". Es kann ferner bevorzugt sein, die Felder mit einer lesbaren Bezeichnung zu versehen, zum Beispiel dem Namen eines Allergens oder einer abgekürzten Bezeichnung, oder mit einem Barcode, Matrixcode, Dotcode oder anderen maschinenlesbaren Bezeichnung.

Es kann ferner bevorzugt sein, mindestens einen Auslesewert, insbesondere Grau- oder Farbwerte als weitere visuelle Kontrolle auf den Träger aufzutragen oder aufzudrucken, die keiner Nachweis- oder Kontrollreaktion unterliegen und auf diese Weise erlauben, einen Vergleichsplatz oder Nachweisplatz zusätzlich mit Referenzwerten zu vergleichen, die Vergleichswerte zu den Auslesewerten unabhängig von jeder Nachweisreaktion liefern.

Das Vergleichsfeld ist beispielsweise durch die vorgegebene Konzentration oder Menge eines Rezeptormoleküls oder Testreagenz, insbesondere eines Antigens oder Antikörpers derart eingestellt, dass seine Helligkeit weder 100% der möglichen Helligkeits- oder Farbänderung am Nachweisplatz beträgt noch 0%. Bevorzugt sind Werte zwischen 20% und 80%, besonders bevorzugt zwischen 30% und 70%, jeweils auf einer linearen oder logarithmischen Skala. Es kann bevorzugt sein, einen Vergleichsplatz mit einem Wert von 50% einzustellen.

Die Erfindung liefert an den besagten Vergleichsfeldern Auslesewerte, die wie die Auslesewerte an den Feldern abhängig von der Führung des Nachweises eine stärkere oder schwächere Veränderung gegenüber dem Ausgangswert aufweisen. Diese gleichsinnige Variation der Auslesewerte ist von Vorteil, da der Nachweis damit tolerant gegen Änderungen in der Nachweisführung wird. Beispielsweise bleibt bei gegenüber der vorschriftsmäßigen Nachweisführung verlängerten oder verkürzten Inkubationszeiten die Vergleichsmöglichkeit zwischen Nachweisplatz (dem Hauptfeld) und Vergleichsfeld erhalten.

Ein weiterer Vorteil besteht darin, dass batch-to-batch Variationen einiger Nachweisreagenzien erfasst werden können. Beispielsweise kann bei einem Antikörper, der mit alkalischer Phosphatase konjugiert ist, eine Variation in seiner apparenten Bindungskonstante, im apparenten Enyzmumsatz, oder in der Qualität des Enzymsubstrats auftreten. In all diesen Fällen variieren die Auslesewerte für Nachweis- und Vergleichsplätze gleichsinnig, und erlauben trotz Variation eine visuelle Bestimmung.

In einer bevorzugten Ausführungsform befinden sich mindestens zwei Vergleichsfelder im selben Blickfeld wie der Nachweisplatz (vorstehend Hauptfeld). Dabei wird der Nachweis bei mindestens zwei Vergleichsfeldern unterschiedlich eingestellt, dass sich die Änderungen der Helligkeit oder der Farbe der Kontrollplätze visuell unterscheiden lassen, wobei das bloße Auge die Änderung der Helligkeit oder Farbe am Nachweisplatz als stärker als das stärkere Kontrollfeld, gleich stark wie das starke Kontrollfeld, zwischen starkem und dem schwächeren Kontrollfeld, gleich schwach wie das schwächere Kontrollfeld, oder schwächer als das schwächere Kontrollfeld, bis hin zu "nicht vorhanden" bestimmen kann.

Bevorzugt ist eine Anordnung, bei der der Nachweisplatz (Hauptfeld) zwischen zwei zugeordneten Kontrollplätzen liegt.

Die Kontrollplätze sind beispielsweise durch vorgegebene Konzentrationen oder Mengen eines Testreagenz, insbesondere eines Antikörpers derart eingestellt, dass ihre Helligkeit jeweils weder 100% der möglichen Helligkeits- oder Farbänderung am Nachweisplatz beträgt noch 0%. Bevorzugt sind bei zwei Kontrollplätzen paarweise Werte von etwa 20% und 80%, besonders bevorzugt von etwa 30% und 70% auf einer linearen oder logarithmischen Skala.

In einer bevorzugten Ausführungsform wird ein Bindungsassay für den Nachweis verwendet, zwischen Rezeptormolekülen und Bindern, wie Peptiden und /oder Proteinen, insbesondere eine Bindung zwischen Antikörper und Antigen, Antikörper und Antikörper, Rezeptor und Ligand, oder DNA, RNA, PNA, LNA und DNA, RNA, PNA, LNA. Die Erfindung schließt den Nachweis ein, bei denen unterschiedliche Moleküle aneinander binden, etwa Aptamere an Proteine, oder PNA an RNA. Es kann vorteilhaft sein, den Nachweis aus mehreren Bindungsreaktionen aufzubauen, etwa durch Verwendung von primären und sekundären Antikörpern.

Detektionsmittel sind sämtliche Nachweismittel und deren Methoden einer Bindungsreaktion.

In einer bevorzugten Ausführungsform ist mindestens eines der Testreagenzien so markiert, dass es auf dem Feld bei positivem Nachweis und auf dem Vergleichsfeld visuell erkannt werden kann. Besonders bevorzugt ist die Markierung mit Gold-Nanopartikeln, Quantum Dots, oder fluoreszierenden Partikeln, unter denen insbesondere Fluoreszenzfarbstoffe bevorzugt sind.

Bei einer weiteren bevorzugten Ausführungsform ist mindestens eines der Testreagenzien mit einem Enzym markiert, und mindestens ein weiteres Testreagenz ein Substrat, das vom Enzym umgesetzt wird und dabei zumindest auf dem Vergleichsplatz und bei positivem Nachweis auch auf dem Nachweisplatz eine mit dem bloßen Auge erkennbare Änderung der Intensität des Auslesewertes hervorruft. Besonders bevorzugt ist diese Ausführungsform eines Nachweises, wenn sie analog zu einem Elisa-Nachweis aufgebaut ist. Dabei ist ein Molekül, beispielsweise ein Antigen oder ein Lebensmittelunverträglichkeits-Antigen, auf dem Nachweisträger fixiert, an den ein Antikörper aus der Probe bindet. An diesen Probenantikörper bindet ein Antikörper aus dem Testreagenz, der mit einem Enzym markiert ist, beispielsweise alkalische Phosphatase. In einem weiteren Schritt wird ein Substrat zugeführt, das vom Enzym umgesetzt wird und dabei zu einer Intensitätsänderung des Auslesewertes führt. Es kann bevorzugt sein das Substrat markiert vorzulegen, beispielsweise mit einem Fluoreszenzfarbstoff, der aufgrund veränderter Wechselwirkung bei Abspaltung eines Teils des Substrats intensiver oder schwächer fluoresziert, oder mit zwei Fluoreszenzfarbstoffen, die sich gegenseitig quenchen und bei Spaltung des Substrat vereinzelt werden und intensiver fluoreszieren, oder zwei Fluoreszenzfarbstoffen, zwischen denen ein Energietransfer stattfindet, und die bei Spaltung des Substrat mit scheinbar geringerem Stokes-Shift vorliegen.

In einer bevorzugten Ausführungsform ist auf dem Feld mindestens ein Antigen fixiert, an das ein Antikörper aus der Probe bindet, an dem wiederum ein markierter Antikörper bindet, und auf einem Vergleichsplatz sind Antikörper fixiert, die nicht aus der Probe stammen, wobei der markierte Antikörper an diese Antikörper bindet.

## Patentansprüche

1. Auswertungseinheit eines Allergietests oder Nahrungsmittelunverträglichkeitstests für eine visuelle Auswertung eines Testergebnisses,
bestehend aus mindestens zwei optisch unterscheidbaren Feldern, **dadurch gekennzeichnet, dass**
mindestens ein erstes Hauptfeld Rezeptormoleküle einer Spezies aufweist,
wobei mindestens ein Allergen Rezeptormoleküle einer Spezies ist,
und mindestens ein weiteres Vergleichsfeld eine geringere Dichte an Rezeptormolekülen einer Spezies aufweist, wobei sämtliche Rezeptormoleküle einer Spezies auf einem Träger fixiert sind,
wobei die Felder für eine Probenflüssigkeit enthaltend einen Binder benetzbar sind,
wobei der Binder mindestens ein Immunglobulin ist,
und der Bindungserfolg mit einem Detektionsmittel nachgewiesen wird, so dass die Auswertungseinheit unterschiedliche Helligkeiten erzeugt.

2. Auswertungseinheit eines Allergietests oder Nahrungsmittelunverträglichkeitstests für eine visuelle Auswertung eines Testergebnisses nach Anspruch 1, wobei Felder mit geringerer Dichte Rezeptormoleküle mit 10 bis 90 Gew. %, insbesondere 20 bis 80 Gew. % an Rezeptormolekülen zum ersten Feld aufweisen.

3. Auswertungseinheit eines Allergietests oder Nahrungsmittelunverträglichkeitstests für eine visuelle Auswertung eines Testergebnisses nach Anspruch 1 oder 2, wobei jedes weitere Feld eine unterschiedliche Dichte an Rezeptormolekülen aufweist.

4. Auswertungseinheit eines Allergietests oder Nahrungsmittelunverträglichkeitstests für eine visuelle Auswertung eines Testergebnisses nach einem der vorhergehenden Ansprüche, wobei die Rezeptormoleküle in den Feldern gleich oder verschieden sind.

5. Auswertungseinheit eines Allergietests oder Nahrungsmittelunverträglichkeitstests für eine visuelle Auswertung eines Testergebnisses nach einem der vorhergehenden Ansprüche, wobei mindestens ein Vergleichsfeld ein Rezeptormolekül einer Spezies aufweist, der mit einem Binder für ein Rezeptormolekül in Hauptfeld identisch ist.

6. Auswertungseinheit eines Allergietests oder Nahrungsmittelunverträglichkeitstests für eine visuelle Auswertung eines Testergebnisses nach einem der vorhergehenden Ansprüche, **dadurch gekennzeichnet, dass** die optisch unterscheidbaren Felder voneinander räumlich getrennt sind, insbesondere können die Felder rund, elliptisch, oval, dreieckig, polygonal, quadratisch oder rechteckig geformt sein, insbesondere in Form eines länglichen Streifens oder Balkens.

7. Auswertungseinheit eines Allergietests oder Nahrungsmittelunverträglichkeitstests für eine visuelle Auswertung eines Testergebnisses nach einem der vorhergehenden Ansprüche, **dadurch gekennzeichnet, dass** der Allergietest oder Nahrungsmittelunverträglichkeitstest, in einem ersten Feld mindestens ein Allergen als Rezeptormoleküle enthält und der Binder mindestens ein Immunglobulin ist, wobei ein weiteres Feld ein Immunglobulin aufweist.

8. Auswertungseinheit eines Allergietests oder Nahrungsmittelunverträglichkeitstests für eine visuelle Auswertung eines Testergebnisses nach einem der vorhergehenden Ansprüche, **dadurch gekennzeichnet, dass** die Probenflüssigkeit eine Körperflüssigkeit, wie Vollblut, Halbblut, EDTA stabilisiertes Blut, Serum, Speichel, Tränenflüssigkeit, Urin oder Gehirnflüssigkeit ist.

9. Allergietests oder Nahrungsmittelunverträglichkeitstests enthaltend ein oder mehrere Auswertungseinheiten nach einem der vorhergehenden Ansprüche, vorzugsweise in Form eines Koordinatensystems.

10. Verfahren zur Diagnose von Allergien und Nahrungsmittelunverträglichkeiten, wobei eine Probenflüssigkeit auf mindestens zwei optisch unterscheidbaren Felder einer Ausleseeinheit aufgetragen wird,
wobei mindestens ein Hauptfeld Rezeptormoleküle einer Spezies aufweist,
wobei mindestens ein Allergen Rezeptormoleküle einer Spezies sind,
und mindestens ein weiteres Vergleichsfeld eine geringere Dichte an Rezeptormolekülen einer Spezies aufweist, wobei sämtliche Rezeptormoleküle einer Spezies auf einem Träger fixiert sind, wobei die Felder mit der Probenflüssigkeit enthaltend einen Binder benetzt werden,
wobei der Binder mindestens ein Immunglobulin ist,
und der Bindungserfolg mit einem Detektionsmittel nachgewiesen wird, so dass die Auswertungseinheit unterschiedliche Helligkeiten erzeugt.

11. Verwendung eines Allergietests oder
Nahrungsmittelunverträglichkeitstests mit einer Ausleseeinheit nach Anspruch 1 im Point-of-Care Bereich.

## Claims

1. An analysis unit of an allergy test or food incompatibility test for visual analysis of a test result, consisting of at least two optically distinguishable fields, **characterized in that**
at least one first main field has receptor molecules of a species,
wherein at least one allergen as receptor molecules of a species,
and at least one further field of comparison has a lower density of receptor molecules of a species,
wherein all receptor molecules of a species are fixed on a carrier,
wherein the fields are wettable for a fluid sample containing a binder,
wherein the binder is at least one immunoglobulin and the binding success is detected with a detection agent so that the analysis unit generates different brightness levels.

2. The analysis unit of an allergy test or food incompatibility test for visual analysis of a test result according to claim 1, wherein fields with a lower density have receptor molecules with 10 to 90% by weight, in particular 20 to 80% by weight of receptor molecules relative to the first field.

3. The analysis unit of an allergy test or food incompatibility test for visual analysis of a test result according to claim 1 or claim 2, wherein each further field has a different density of receptor molecules.

4. The analysis unit of an allergy test or food incompatibility test for visual analysis of a test result according to any one of the preceding claims, wherein the receptor molecules in the fields are the same or different.

5. The analysis unit of an allergy test or food incompatibility test for visual analysis of a test result according to any one of the preceding claims, wherein at least one reference field has a receptor molecule of a species which is identical to a binder for a receptor molecule in the main field.

6. The analysis unit of an allergy test or food incompatibility test for visual analysis of a test result according to any one of the preceding claims, **characterized in that** the optically distinguishable fields are spatially separated from each other and, in particular, the fields can be formed round, elliptical, oval, triangular, polygonal, square or rectangular, in particular in the form of an elongated strip or bar.

7. The analysis unit of an allergy test or food incompatibility test for visual analysis of a test result according to any one of the preceding claims, **characterized in that** the allergy test or the food incompatibility test contains in a first field at least one allergen as receptor molecules and the binder is at least one immunoglobulin, wherein optionally, a further field has an immunoglobulin.

8. The analysis unit of an allergy test or food incompatibility test for visual analysis of a test result according to any one of the preceding claims, **characterized in that** the sample fluid is a body fluid such as whole blood, half blood, EDTA stabilized blood, serum, saliva, lacrimal fluid, urine or brain fluid.

9. Allergy test or food incompatibility test containing one or a plurality of analysis units according to any one of the preceding claims, preferably in the form of a coordinate system.

10. A method for diagnosing allergies and food incompatibilities, wherein a sample fluid is applied onto at least two optically distinguishable fields of a readout unit,
wherein at least one main field has receptor molecules of a species,
wherein at least one allergen as receptor molecules of a species,
and at least one further field of comparison has a lower density of receptor molecules of a species, wherein all receptor molecules of a species are fixed on a carrier, wherein the fields are wetted with the sample fluid containing a binder,
wherein the binder is at least one immunoglobulin and the binding success is detected with a detection agent so that the analysis unit generates different brightness levels.

11. Use of an allergy test or food incompatibility test with a readout unit according to claim 1 in the point-of-care testing field.

## Revendications

1. Unité d'évaluation d'un test d'allergie ou de tests de tolérance à des produits alimentaires permettant une évaluation visuelle du résultat du test, constitué d'au moins deux champs pouvant être différentiés optiquement, **caractérisée en ce qu'**au moins un premier champ principal présente des molécules de récepteurs d'une espèce,
où au moins un allergène est une molécule de récepteur d'une espèce,
et au moins un autre champ de comparaison présente une densité moins importante en molécules de récepteurs d'une espèce,
où toutes les molécules de récepteurs d'une espèce sont fixées sur un support,
où les champs peuvent être mouillés avec un liquide de test contenant un liant,
où le liant est au moins une immunoglobuline,
et la réussite de la liaison est évaluée avec un produit de détection de sorte que l'unité d'évaluation génère des éclairages différents.

2. Unité d'évaluation d'un test d'allergie ou de tests de tolérance à des produits alimentaires permettant une évaluation visuelle du résultat du test selon la revendication 1, dans laquelle des champs avec une densité de molécules de récepteurs moindre présentent de 10 à 90 % en poids, notamment de 20 à 80 % en poids, de molécules de récepteurs par rapport au premier champ.

3. Unité d'évaluation d'un test d'allergie ou de tests de tolérance à des produits alimentaires permettant une évaluation visuelle du résultat du test selon la revendication 1 ou la revendication 2, dans laquelle chaque nouveau champ présente une densité en molécules de récepteurs différente.

4. Unité d'évaluation d'un test d'allergie ou de tests de tolérance à des produits alimentaires permettant une évaluation visuelle du résultat du test selon l'une des revendications précédentes, dans laquelle les molécules de récepteurs sont identiques ou différentes dans les champs.

5. Unité d'évaluation d'un test d'allergie ou de tests de tolérance à des produits alimentaires permettant une évaluation visuelle du résultat du test selon l'une des revendications précédentes, dans laquelle au moins un champ de comparaison présente une molécule de récepteur d'une espèce, qui est identique avec un liant pour une molécule de récepteur dans le champ principal.

6. Unité d'évaluation d'un test d'allergie ou de tests de tolérance à des produits alimentaires permettant une évaluation visuelle du résultat du test selon l'une des revendications précédentes, **caractérisée en ce que** les champs pouvant être différentiés optiquement sont séparés les uns des autres dans l'espace, notamment, les champs peuvent être sous forme circulaire, elliptique, ovale, triangulaire, polygonale, carrée ou rectangulaire, notamment sous la forme d'une bande allongée ou d'une poutre.

7. Unité d'évaluation d'un test d'allergie ou de tests de tolérance à des produits alimentaires permettant une évaluation visuelle du résultat du test selon l'une des revendications précédentes, **caractérisée en ce que** le test d'allergie ou de tolérance de produit alimentaire contient, dans un premier champ, au moins un allergène en tant que molécule de récepteur et le liant est au moins une immunoglobuline, où un autre champ présente une immunoglobuline.

8. Unité d'évaluation d'un test d'allergie ou de tests de tolérance à des produits alimentaires permettant une évaluation visuelle du résultat du test selon l'une des revendications précédentes, **caractérisée en ce que** le liquide de test est un liquide corporel, tel que du sang entier, du sang hybride, du sang stabilisé à l'EDTA, du sérum, de la salive, des larmes, de l'urine ou du liquide cervical.

9. Tests d'allergie ou tests de tolérance de produits alimentaires contenant une ou plusieurs unités d'évaluation selon l'une des revendications précédentes, de préférence sous la forme d'un système de coordonnées.

10. Procédé de diagnostic d'allergies et de tolérances de produits alimentaires, dans lequel un liquide de test est déposé sur au moins deux champs pouvant être différentiés optiquement d'une unité de lecture,
où au moins un champ principal présente des molécules de récepteurs d'une espèce,
où au moins un allergène est une molécule de récepteur d'une espèce,
et au moins un autre champ de comparaison présente une densité plus faible en molécules de récepteurs d'une espèce, où toutes les molécules de récepteurs sont fixées sur un support, où les champs avec le liquide de test contenant un liant sont mouillées,
où le liant est au moins une immunoglobuline, et la réussite de la liaison est évaluée avec un produit de détection de sorte que l'unité d'évaluation génère des éclairages différents.

11. Utilisation d'un test d'allergie ou d'un test de tolérance à un produit alimentaire doté d'une unité de lecture selon la revendication 1 dans le domaine du lieu d'intervention.
